# EUROPEAN PATENT APPLICATION

(11) **EP 4 840 397 A1**
(43) Date of publication of application: **18.11.2026**
(21) Application number: 25755121.8
(22) Date of filing: 11.02.2025
(51) Int. Cl.: A61B 5/1455, A61B 5/00, A61B 5/145

(54) **WEARABLE DEVICE AND METHOD FOR MEASURING SKIN FLUORESCENCE USING SAME**

(30) Priority: 13.02.2024 KR 20240020455; 05.04.2024 KR 20240046478
(71) Applicant: Samsung Electronics Co., Ltd., Suwon-si, Gyeonggi-do 16677 (KR)
(72) Inventor: CHO, Minhyun, Suwon-si Gyeonggi-do 16677 (KR); SHIN, Euiseok, Suwon-si Gyeonggi-do 16677 (KR); JUNG, Hyunjoo, Suwon-si Gyeonggi-do 16677 (KR); KIM, Taeseon, Suwon-si Gyeonggi-do 16677 (KR); PARK, Minho, Suwon-si Gyeonggi-do 16677 (KR); AHN, Joongwoo, Suwon-si Gyeonggi-do 16677 (KR)
(74) Representative: Gulde & Partner
(86) International application number: PCT/KR2025/001958
(87) International publication number: WO 2025/174020

(57) **Abstract**

A wearable device is provided. The wearable device may include a housing including a light transmission area, a board disposed inside the housing and including a surface directed toward the light transmission area, a first photodetector disposed on the surface of the board and configured to detect light having a wavelength equal to or longer than a first wavelength, a second photodetector disposed on the surface of the board and configured to detect light having a wavelength equal to or longer than a second wavelength longer than the first wavelength, a first light emitter disposed between the first photodetector and the second photodetector and configured to emit light having a third wavelength toward the light transmission area, and a second light emitter disposed between the first light emitter and the second photodetector and configured to emit light having a fourth wavelength shorter than the third wavelength toward the light transmission area.

## Description

### TECHNICAL FIELD

The disclosure relates to an electronic device. More particularly, the disclosure relates to a wearable device including a light emitter and a photodetector and a method for measuring skin fluorescence using the same.

### BACKGROUND ART

An electronic device may refer to a device that executes a specific function according to a loaded program, such as a home appliance, an electronic notebook, a portable multimedia player, a mobile communication terminal, a tablet personal computer (PC), a video/audio device, a desktop/laptop computer, or a vehicle navigation device. For example, these electronic devices may output stored information as sound or an image.

As the integration level of electronic devices increases and high-speed, large-capacity wireless communication becomes more common, a single electronic device such as a mobile communication terminal may recently be equipped with various functions. For example, an entertainment function such as games, a multimedia function such as music/video playback, a communication and security function for mobile banking, and a function such as schedule management or an electronic wallet in addition to a communication function are integrated into a single electronic device. Such electronic devices are miniaturized so that users may carry them conveniently.

The above information is presented as background information only to assist with an understanding of the disclosure. No determination has been made, and no assertion is made, as to whether any of the above might be applicable as prior art with regard to the disclosure.

### DISCLOSURE OF INVENTION

### Solution to Problems

Aspects of the disclosure are to address at least the above-mentioned problems and/or disadvantages and to provide at least the advantages described below. Accordingly, an aspect of the disclosure is to provide a wearable device including a light emitter and a photodetector and a method for measuring skin fluorescence using the same.

Additional aspects will be set forth in part in the description which follows and, in part, will be apparent from the description, or may be learned by practice of the presented embodiments.

In accordance with an aspect of the disclosure, a wearable device is provided. The wearable device includes a housing including a light transmission area, a board disposed inside the housing and including a surface directed toward the light transmission area, a first photodetector disposed on the surface of the board and configured to detect light having a wavelength equal to or longer than a first wavelength, a second photodetector disposed on the surface of the board and configured to detect light having a wavelength equal to or longer than a second wavelength longer than the first wavelength, a first light emitter disposed between the first photodetector and the second photodetector and configured to emit light having a third wavelength toward the light transmission area, and a second light emitter disposed between the first light emitter and the second photodetector and configured to emit light having a fourth wavelength shorter than the third wavelength toward the light transmission area.

In accordance with another aspect of the disclosure, a method performed by a wearable device for measuring skin fluorescence including a light emission module configured to emit light toward a user's skin and a photodetecting module configured to detect light emitted from the user's skin is provided. The method includes emitting, by the wearable device, at least one of red light, blue light, or green light toward the user's skin through the light emission module and obtaining a skin color property signal value through the photodetecting module, emitting, by the wearable device, an infrared (IR) light toward the user's skin through the light emission module and obtaining a skin thickness property signal value through the photodetecting module, setting, by the wearable device, an illuminance of an ultraviolet (UV) light emitted through the light emission module based on the obtained skin color property signal value and the obtained skin thickness property signal value, and emitting, by the wearable device, the UV light with the set illuminance toward the user's skin through the light emission module and obtaining a skin fluorescence signal value through the photodetecting module.

In accordance with another aspect of the disclosure, one or more non-transitory computer-readable storage media storing one or more computer programs including computer-executable instructions that, when executed by one or more processors of a wearable device individually or collectively, cause the wearable device to perform operations are provided. The operations include emitting, by the wearable device, at least one of red light, blue light, or green light toward a user's skin through a light emission module and obtaining a skin color property signal value through a photodetecting module, emitting, by the wearable device, infrared (IR) light toward the user's skin through the light emission module and obtaining a skin thickness property signal value through the photodetecting module, setting, by the wearable device, an illuminance of ultraviolet (UV) light emitted through the light emission module based on the obtained skin color property signal value and the obtained skin thickness property signal value; and emitting, by the wearable device, UV light with the set illuminance toward the user's skin through the light emission module and obtaining a skin fluorescence signal value through the photodetecting module.

Other aspects, advantages, and salient features of the disclosure will become apparent to those skilled in the art from the following detailed description, which, taken in conjunction with the annexed drawings, discloses various embodiments of the disclosure.

### BRIEF DESCRIPTION OF DRAWINGS

The above and other aspects, features, and advantages of certain embodiments of the disclosure will be more apparent from the following description taken in conjunction with the accompanying drawings, in which:
FIG. 1 is a block diagram illustrating an electronic device in a network environment according to an embodiment of the disclosure;
FIG. 2 is a perspective view of a wearable device, illustrating a front surface of the wearable device according to an embodiment of the disclosure;
FIG. 3 is a perspective view of a wearable device illustrating a rear surface of the wearable device according to an embodiment of the disclosure;
FIG. 4 illustrates an example in which a wearable device according to an embodiment of the disclosure is worn on a user's wrist;
FIG. 5 is a cross-sectional view illustrating a user's skin composed of an epidermal layer and a dermal layer, referred to for describing the principle of skin fluorescence measurement of a wearable device according to an embodiment of the disclosure;
FIG. 6 illustrates light emitters and photodetectors of a sensor module according to an embodiment of the disclosure;
FIG. 7 is a cross-sectional view illustrating a portion of a wearable device and sensor module according to an embodiment of the disclosure;
FIG. 8 illustrates distances between light emitters and photodetectors of a sensor module according to an embodiment of the disclosure;
FIG. 9 illustrates a plurality of light emission modules and photodetectors according to an embodiment of the disclosure;
FIG. 10 illustrates first light emission modules and photodetectors according to an embodiment of the disclosure;
FIG. 11 illustrates light emitters and photodetectors according to an embodiment of the disclosure;
FIG. 12 illustrates light emission modules and photodetectors according to an embodiment of the disclosure;
FIG. 13 is a block diagram illustrating a portion of a wearable device according to an embodiment of the disclosure;
FIG. 14 is a flowchart illustrating a method for measuring skin fluorescence using a wearable device according to an embodiment of the disclosure; and
FIG. 15 is a flowchart illustrating an operation of obtaining a skin fluorescence signal value according to an embodiment of the disclosure.

The same reference numerals are used to represent the same elements throughout the drawings.

### MODE FOR THE INVENTION

The following description with reference to the accompanying drawings is provided to assist in a comprehensive understanding of various embodiments of the disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the claims and their equivalents.

Unless the context clearly dictates otherwise, the singular forms "a", "an", and "the" should be understood to include plural meanings. Therefore, for example, a "surface of a component" may be understood to include one or more of the surfaces of the component.

It should be appreciated that the blocks in each flowchart and combinations of the flowcharts may be performed by one or more computer programs which include instructions. The entirety of the one or more computer programs may be stored in a single memory device or the one or more computer programs may be divided with different portions stored in different multiple memory devices.

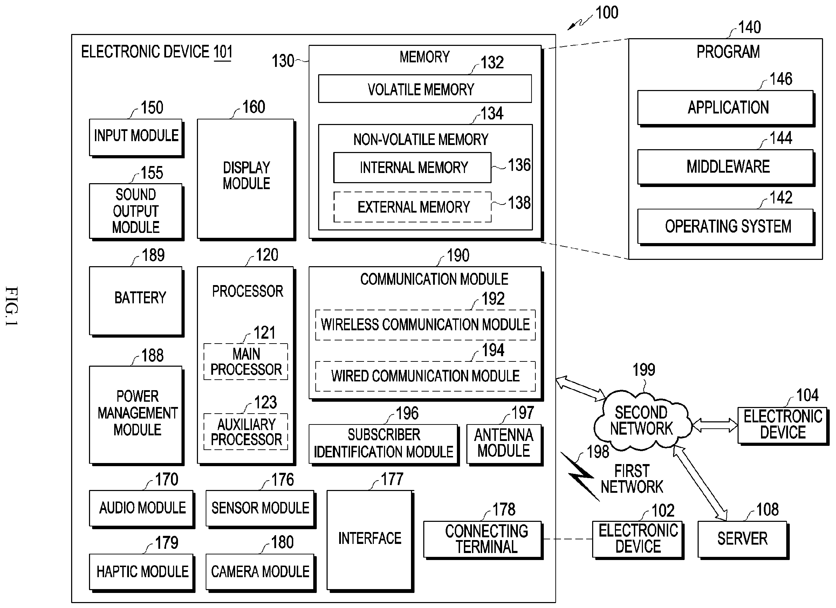

FIG. 1 is a block diagram illustrating an electronic device 103 in a network environment 100 according to an embodiment of the disclosure.

Referring to FIG. 1, an electronic device 101 in a network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may include various processing circuitry and/or multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions.

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the strength of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a fifth generation (5G) network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a fourth generation (4G) network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the millimeter wave (mmWave) band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form an mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102 or 104, or the server 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. As used herein, each of such phrases as "A or B", "at least one of A and B", "at least one of A or B", "A, B, or C", "at least one of A, B, and C", and "at least one of A, B, or C", may include any one of, or all possible combinations of the items enumerated together in a corresponding one of the phrases. As used herein, such terms as "1st" and "2nd", or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with", "coupled to", "connected with", or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, logic, logic block, part, or circuitry. A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., the internal memory 136 or the external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

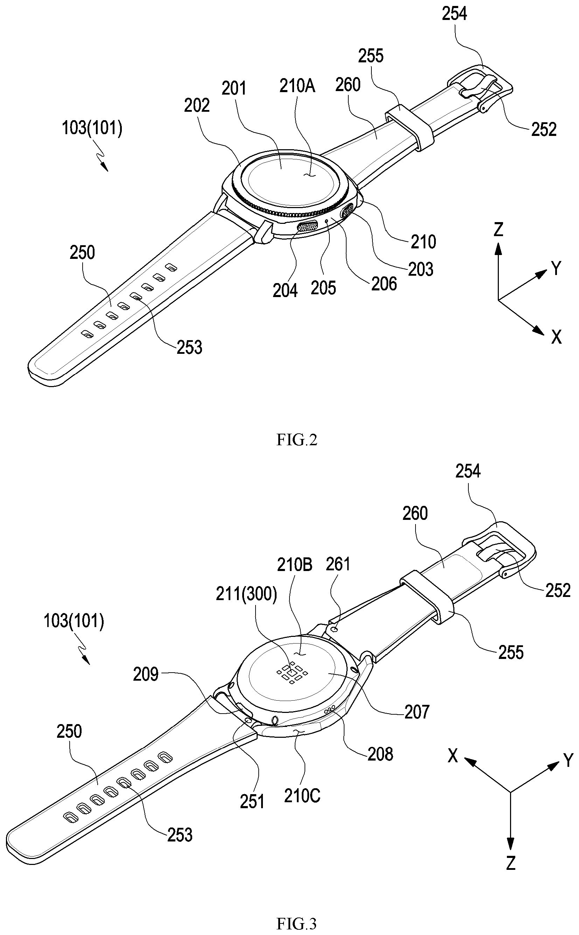

FIG. 2 is a perspective view of a wearable device 103, illustrating a front surface of the wearable device according to an embodiment of the disclosure. FIG. 3 is a perspective view of a wearable device 103, illustrating a rear surface of the wearable device 103 according to an embodiment of the disclosure.

The description of the electronic device 101 made with reference to FIG. 1 is applicable substantially equally to the wearable device 103 described with reference to FIGS. 2 to 15, unless conflicting each other.

Referring to FIGS. 2 and 3, the wearable device 103 (e.g., the electronic device 101 in FIG. 1) according to an embodiment of the disclosure may include a housing 210 or wearing members 250 and 260. The housing 210 may include a first surface (or front surface) 210A, a second surface (or rear surface) 210B, or a side surface 210C. The side surface 210C may surround a space between the first surface 210A and the second surface 210B.

According to an embodiment of the disclosure, the housing 210 may refer to a structure that forms a portion of the first surface 210A of FIG. 2 and the second surface 210B and side surface 210C of FIG. 3. At least a portion of the first surface 210A may be formed by a front plate 201 (e.g., a glass plate or polymer plate including various coating layers) which is at least partially substantially transparent. The second surface 210B may be formed by a substantially opaque rear plate 207. The housing 210 may include the front plate 201, a side bezel structure 206, and/or the rear plate 207.

According to an embodiment of the disclosure, the rear plate 207 may include an at least partially transparent area. Light may pass from the outside to the inside of the wearable device 103 through the transparent area, and light may be emitted from the inside of the wearable device 103 to the outside. Accordingly, the transparent area may be referred to as a light transmission area.

According to an embodiment of the disclosure, the rear plate 207 may be formed of, for example, coated or tinted glass, ceramic, a polymer, a metal (e.g., aluminum, stainless steel (STS), or magnesium), or a combination of at least two of these materials. The side surface 210C may be formed by the side bezel structure (or "side member") 206 coupled to the front plate 201 and the rear plate 207 and including a metal and/or a polymer. In an embodiment, the rear plate 207 and the side bezel structure 206 may be integrally formed and include the same material (e.g., a metallic material such as aluminum).

According to an embodiment of the disclosure, the wearable device 103 may include at least one of a display (e.g., the display module 160 in FIG. 1), audio modules 205 and 208 (e.g., the audio module 170 in FIG. 1), a sensor module 211 (e.g., the sensor module 176 in FIG. 1), key input devices 202, 203 and 204 (e.g., the input module 150 in FIG. 1), or a connector hole 209 (e.g., the connecting terminal 178 in FIG. 1). In an embodiment, the wearable device 103 may not be provided with at least one (e.g., the key input devices 202, 203 and 204, the connector hole 209, or the sensor module 211) of the components or additionally include other components.

According to an embodiment of the disclosure, the display may be exposed, for example, through a substantial portion of the front plate 201. The shape of the display may correspond to that of the front plate 201, and may be in any of various shapes such as circle, oval, or polygon. The display may be incorporated with or disposed adjacent to a touch sensing circuit, a pressure sensor that measures the intensity (pressure) of a touch, and/or a fingerprint sensor.

According to an embodiment of the disclosure, the audio modules 205 and 208 may include a microphone hole 205 and a speaker hole 208. A microphone for obtaining an external sound may be disposed in the microphone hole 205, and in some embodiments, a plurality of microphones may be disposed to detect the direction of a sound. The speaker hole 208 may be used as an external speaker and a receiver for calls. In an embodiment, a speaker (e.g., a piezo speaker) may be included without the speaker hole.

According to an embodiment of the disclosure, the key input devices 202, 203, and 204 may include a wheel key 202 disposed on the first surface 210A of the housing 210 and rotatable in at least one direction, and/or side key buttons 203 and 204 disposed on the side surface 210C of the housing 210. The wheel key 202 may have a shape corresponding to that of the front plate 201. In an embodiment, the wearable device 103 may not include some or any of the above-mentioned key input devices 202, 203, and 204, and the key input devices 202, 203, and 204 which are not included may be implemented in other forms such as soft keys on the display. The connector hole 209 may accommodate a connector (e.g., a USB connector) for transmitting and receiving power and/or data to and from an external electronic device, and include another connector hole (not shown) for accommodating a connector for transmitting and receiving an audio signal to and from an external electronic device. The wearable device 103 may further include, for example, a connector cover (not shown) that covers at least a portion of the connector hole 109 and blocks the introduction of a foreign material into the connector hole 109.

According to an embodiment of the disclosure, the wearable device 103 may be configured to be detachably fastened to a user's body part (e.g., wrist, ankle, or the like) through the wearing members 250 and 260. The wearing members 250 and 260 may be connected to at least a portion of the housing 210. The wearing members 250 and 260 may be formed of various materials in various shapes. An integrated link and a plurality of unit links may be formed of fabric, leather, rubber, urethane, metal, ceramic, or a combination of at least two of these materials, to be movable together.

According to an embodiment of the disclosure, the wearing members 250 and 260 may be detachably fastened to at least a partial area of the housing 210 using locking members 251 and 261. The locking members 251 and 261 may include fastening components, such as pogo pins, and may be replaced by protrusion(s) or recess(es) formed on the wear members 250 and 260 according to an embodiment. For example, the wearing members 250 and 260 may be coupled in such a manner that they are engaged with grooves or protrusions formed on the housing 210. The wearing members 250 and 260 may include one or more of a fixing member 252, a fixing member fastening hole 253, a band guide member 254, and a band fixing loop 255.

According to an embodiment of the disclosure, the fixing member 252 may be configured to fix the housing 210 and the wearing members 250 and 260 to the user's body part (e.g., wrist or ankle). The fixing member fastening hole 253 may fix the housing 210 and the wearing members 250 and 260 to the user's body part in correspondence with the fixing member 252. The band guide member 254 may be configured to limit a movement range of the fixing member 252, when the fixing member 252 is fastened in the fixing member fastening hole 253, so that the wearing members 250 and 260 are fastened to the user's body part in close contact. The band fixing loop 255 may limit movement ranges of the wearing members 250 and 260, with the fixing member 252 fastened in the fixing member fastening hole 253.

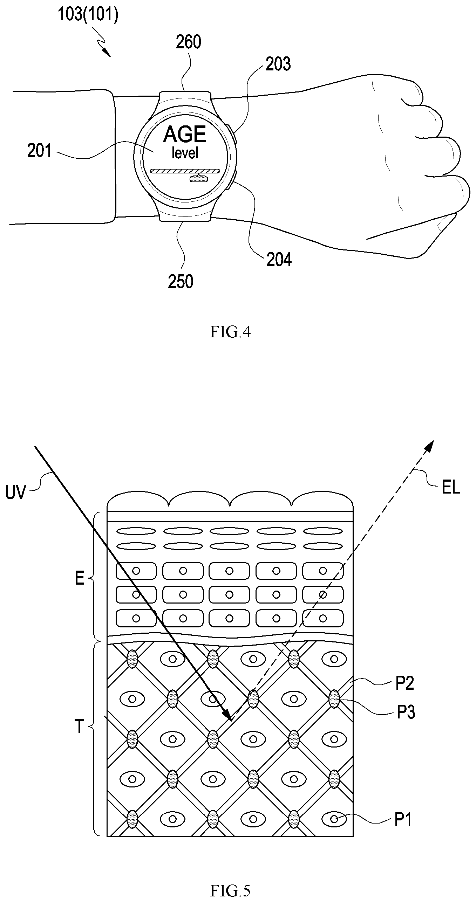

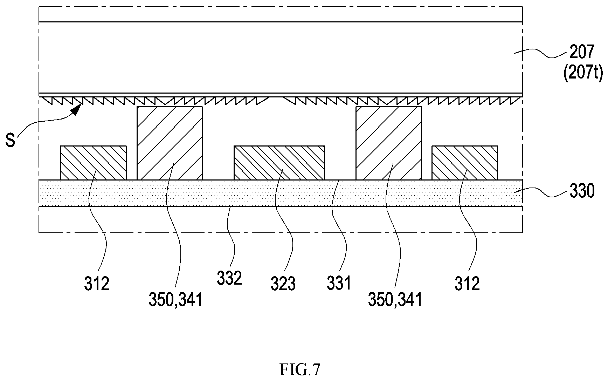

According to an embodiment of the disclosure, the sensor module 211 or 300 may measure biometric information about the user through a light transmission area 207t (see FIG. 7) of the rear plate 207 directed toward the user's body. The sensor module 211 or 300 may include, for example, a biometric sensor module 211 or 300 disposed on the second surface 210B of the housing 210. The sensor module 211 or 300 will be described in detail later with reference to FIGS. 4 to 15.

FIG. 4 illustrates an example in which a wearable device 103 is worn on the user's wrist according to an embodiment of the disclosure.

Referring to FIG. 4, the wearable device 103 according to an embodiment of the disclosure may be worn on the user's body part (e.g., wrist). The wearable device 103 may be worn on the user's body such that the rear plate 207 (see FIG. 3) is directed toward the user's skin and the front plate 201 is directed toward the outside of the user's body part. Data measured (or detected) by the wearable device 103 may be displayed on the display to provide information to the user. In addition, the sensor module 211 or 300 (see FIG. 3) disposed on the rear plate 207 may measure biometric information about the user by emitting light to the user's skin through the rear plate 207 and detecting reflected light of the emitted light or light (e.g., light absorbed into and emitted by skin proteins) generated by the emitted light.

FIG. 5 is a cross-sectional view illustrating a user's skin composed of an epidermal layer E and a dermal layer T to describe the principle of skin fluorescence measurement of a wearable device 103 according to an embodiment of the disclosure. Excess glucose or fructose in the body may be combined with dermal layer proteins P1, P2, and P3 of the dermal layer T to produce an advanced glycated end products (AGEs). The AGEs may be accumulated in the dermal layer proteins P1, P2, and P3. For example, the dermal layer proteins P1, P2, and P3 may include hyaluronic acid P1, collagen P2, elastin P3, and so on.

Referring to FIG. 5, according to an embodiment of the disclosure, light UV emitted from the sensor module 300 toward the user's skin may reach the epidermal layer E and dermal layer T of the skin. For example, the light UV emitted toward the user's skin may be ultraviolet (UV) light. A portion of the emitted light UV may be absorbed by the dermal layer proteins P1, P2, and P3 with the combined AGEs, located in the dermal layer T, and emitted to the outside of the skin in the form of fluorescence. As the energy of the emitted light UV is absorbed by the dermal layer proteins P1, P2, and P3, the wavelength of the emitted light EL may be longer than that of the emitted light UV. The light EL emitted in the form of fluorescence from the dermal layer proteins P1, P2, and P3 may be detected by the sensor module 300.

According to an embodiment of the disclosure, the wearable device 103 may calculate the amount of the dermal layer proteins P1, P2, and P3 in which the AGEs are accumulated, based on the wavelength of the light UV emitted toward the user's skin, the wavelength of the emitted light EL, and the amount (illuminance) of the emitted light EL. Accordingly, the amount of the AGEs in the body may be measured. The measurement of AGEs described with reference to FIG. 5 may be understood as an example of applying the skin fluorescence measurement method of the wearable device 103 101 according to an embodiment of the disclosure.

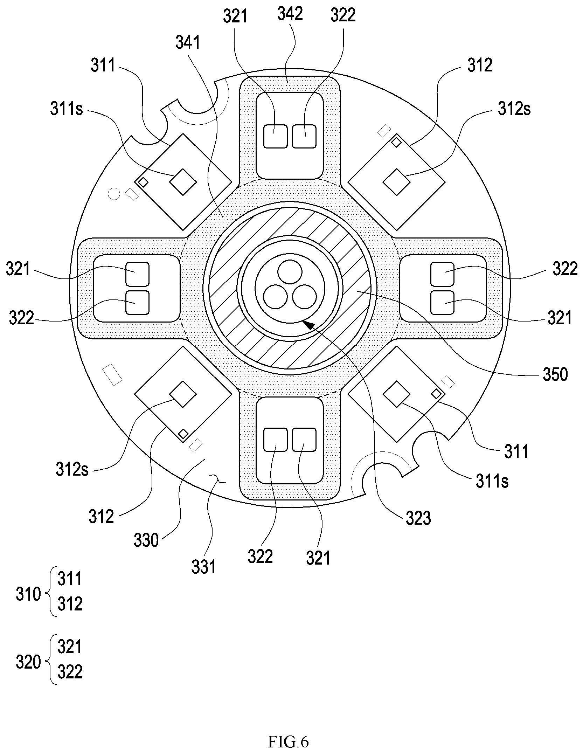

FIG. 6 illustrates light emitters 321 and 322 and photodetectors 311 and 312 in a sensor module 300 according to an embodiment of the disclosure.

Referring to FIG. 6, the wearable device 103 according to an embodiment of the disclosure may include a board 330 disposed inside the housing 210. The board 330 may include a plate on which, for example, light emitters 321, 322, and 323 and/or the photodetectors 311 and 312 are disposed, or a PCB on which the light emitters 321, 322, and 323 and/or the photodetectors 311 and 312 are mounted by surface mounted technology (SMT). The board 330 may also be referred to as a plate or PCB.

According to an embodiment of the disclosure, the board 330 may include a surface 331 directed toward the light transmission area 207t of the housing 210 (see FIG. 2). In an example, the surface 331 of the board 330 may face the light transmission area 207t formed on the rear plate 207. The board 330 may have a shape corresponding to the light transmission area 207t of the housing 210. In an example, when viewed from the outside of the light transmission area 207t, the board 330 and the light transmission area 207t may at least partially overlap.

According to an embodiment of the disclosure, the wearable device 103 may include a photodetecting module 310 configured to detect light reflected or emitted from the user's skin. The photodetecting module 310 may include a first photodetector 311. The first photodetector 311 may be disposed on the surface 331 of the board 330. The first photodetector 311 may be configured to detect (or receive) light in a band of a first wavelength or longer. In an example, the first photodetector 311 may detect light in a band of 430nm or longer. In an example, the first photodetector 311 may include a photodiode.

According to an embodiment of the disclosure, a plurality of first photodetectors 311 may be provided. In an example, the plurality of first photodetectors 311 may be arranged on the surface 331 of the board 330 along an edge of the surface 331 of the board 330. In another example, the plurality of first photodetectors 311 may be arranged on the surface 331 of the board 330 along an edge of the light transmission area 207t.

According to an embodiment of the disclosure, the photodetecting module 310 may include a second photodetector 312. The second photodetector 312 may be disposed on the surface 331 of the board 330. The second photodetector 312 may be spaced apart from the first photodetector 311. The second photodetector 312 may be configured to detect (or receive) light in a band of a wavelength equal to or longer than a second wavelength longer than the first wavelength. In an example, the second photodetector 312 may include a photodiode equipped with a filter that passes light having a specific wavelength or longer (e.g., 500nm or longer), to measure the amount of light corresponding to the wavelength of the emitted light EL (see FIG. 5).

According to an embodiment of the disclosure, a plurality of second photodetectors 312 may be provided. In an example, the plurality of second photodetectors 312 may be arranged on the surface 331 of the board 330 along the edge of the surface 331 of the board 330. In another example, the plurality of second photodetectors 312 may be arranged on the surface 331 of the board 330 along the edge of the light transmission area 207t.

The wearable device 103 according to an embodiment of the disclosure may include a first light emitter 321. In an example, the first light emitter 321 may include an LED. The first light emitter 321 may be configured to emit light having a third wavelength toward the light transmission area 207t. In an example, the first light emitter 321 may emit blue light. A band of the third wavelength used by the first light emitter 321 may be included in a wavelength band between 430nm and 480nm.

According to an embodiment of the disclosure, the first light emitter 321 may be disposed on the surface 331 of the board 330. The first light emitter 321 may be spaced apart from the first photodetector 311 and the second photodetector 312. The first light emitter 321 may be located between the first photodetector 311 and the second photodetector 312.

According to an embodiment of the disclosure, a plurality of first light emitters 321 may be provided. In an example, the plurality of first light emitters 321 may be arranged on the surface 331 of the board 330 along the edge of the surface 331 of the board 330. In another example, the plurality of first light emitters 321 may be arranged on the surface 331 of the board 330 along the edge of the light transmission area 207t.

The wearable device 103 according to an embodiment of the disclosure may include a second light emitter 322. In an example, the second light emitter 322 may include an LED. The second light emitter 322 may be configured to emit light having a fourth wavelength shorter than the third wavelength toward the light transmission area 207t. In an example, the first light emitter 321 may emit blue light, and the second light emitter 322 may emit UV light.

According to an embodiment of the disclosure, the first light emitter 321 and the second light emitter 322 may be referred to as a first light emission module 320. When the second light emitter 322 operates, the first light emitter 321 may also operate. Accordingly, signal values obtained through the photodetectors 311 and 312 may be corrected by emitting light through the second light emitter 322 with the first light emitter 321.

According to an embodiment of the disclosure, the second light emitter 322 may be disposed on the surface 331 of the board 330. The second light emitter 322 may be spaced apart from the first photodetector 311 and the second photodetector 312. The second light emitter 322 may be located between the second photodetector 312 and the first light emitter 321. In an example, the first photodetector 311, the first light emitter 321, the second light emitter 322, and the second photodetector 312 may be sequentially arranged along the edge of the surface of the board 330.

According to an embodiment of the disclosure, a plurality of second light emitters 322 may be provided. In an example, the plurality of second light emitters 322 may be arranged on the surface 331 of the board 330 along the edge of the surface 331 of the board 330. In another example, the plurality of second light emitters 322 may be arranged on the surface 331 of the board 330 along the edge of the light transmission area 207t.

The wearable device 103 according to an embodiment of the disclosure may include a second light emission module 323 configured to emit light having a fifth wavelength longer than the third wavelength toward the light transmission area 207t. The second light emission module 323 may include a plurality of third light emitters 323a, 323b, and 323c. The second light emission module 323 may be disposed on the surface 331 of the board 330.

According to an embodiment of the disclosure, the plurality of third light emitters 323a, 3223b, and 323c may be configured to emit light having a wavelength equal to or longer than the third wavelength. The plurality of third light emitters 323a, 3223b, and 323c may include an IR light emitter 323a configured to emit IR light. A band of the fourth wavelength used by the IR light emitter 323a may be included in a wavelength band of 780nm or longer. The plurality of third light emitters 323a, 3223b, and 323c may include a red light emitter 323b configured to emit red light. A wavelength band used by the red light emitter 323b may be included in a wavelength band between 645nm and 700nm. The plurality of third light emitters 323a, 3223b, and 323c may include a green light emitter 323c configured to emit green light. A wavelength band used by the green light emitter 323c may be included in a wavelength band between 490nm and 530nm. In an example, the second light emission module 323 may include at least one of the IR light emitter 323a, the red light emitter 323b, or the green light emitter 323c.

According to an embodiment of the disclosure, the first photodetector 311, the second photodetector 312, the first light emitter 321, and the second light emitter 322 may be arranged around the second light emission module 323 to at least partially surround the second light emission module 323. In an example, the first photodetector 311, the second photodetector 312, the first light emitter 321, and the second light emitter 322 may be arranged at radial positions of the second light emission module 323.

According to an embodiment of the disclosure, the wearable device 103 may include a first wall 350. The first wall 350 may protrude from the surface 331 of the board 330 toward the light transmission area 207t. The first wall 350 may surround the second light emission module 323. The first wall 350 may be disposed between the third light emitters 323a, 3223b, and 323c and the first photodetector 311. The first wall 350 may be disposed between the third light emitters 323a, 3223b, and 323c and the second photodetector 312. The first wall 350 may be disposed between the third light emitters 323a, 3223b, and 323c and the first light emitter 321. The first wall 350 may be disposed between the third light emitters 323a, 3223b, and 323c and the second light emitter 322.

According to an embodiment of the disclosure, the wearable device 103 may include a second wall 341 and 342. The second wall 341 and 342 may protrude from the surface 331 of the board 330 toward the light transmission area 207t. The second wall 341 and 342 may include a first portion 341 and a second portion 342.

According to an embodiment of the disclosure, the first portion 341 of the second wall 341 and 342 may surround the first wall 350. The second portion 342 of the second wall 341 and 342 may at least partially surround the first light emission module 320. In an example, the first portion 341 and the second portion 342 of the second wall 341 and 342 may surround the first light emission module 320 together.

According to an embodiment of the disclosure, the first portion 341 of the second wall 341 and 342 may be disposed between the third light emitters 323a, 3223b and 323c and the first photodetector 311. The first portion 341 of the second wall 341 and 342 may be disposed between the third light emitters 323a, 3223b, and 323c and the second photodetector 312.

According to an embodiment of the disclosure, the second portion 342 of the second wall 341 and 342 may be disposed between the first photodetector 311 and the first light emitter 321. The second portion 342 of the second wall 341 and 342 may be disposed between the second photodetector 312 and the second light emitter 322.

FIG. 7 is a cross-sectional view illustrating a portion of a wearable device 103 and a sensor module 300 according to an embodiment of the disclosure.

Referring to FIGS. 6 and 7, according to an embodiment of the disclosure, the light transmission area 207t of the rear plate 207 may include a serration pattern S. Light emitted from the light emitters 321 and 322 may easily reach the user's body part by the serration pattern S. Due to the serration pattern S, light from the outside of the wearable device 103 may easily reach the photodetectors 311 and 312 through the light transmission area 207t. The walls 341, 342, and 350 may protrude from one surface of the board 330 toward the light transmission area 207t. The other surface 332 of the board 330 may be directed toward the inside of the wearable device 103.

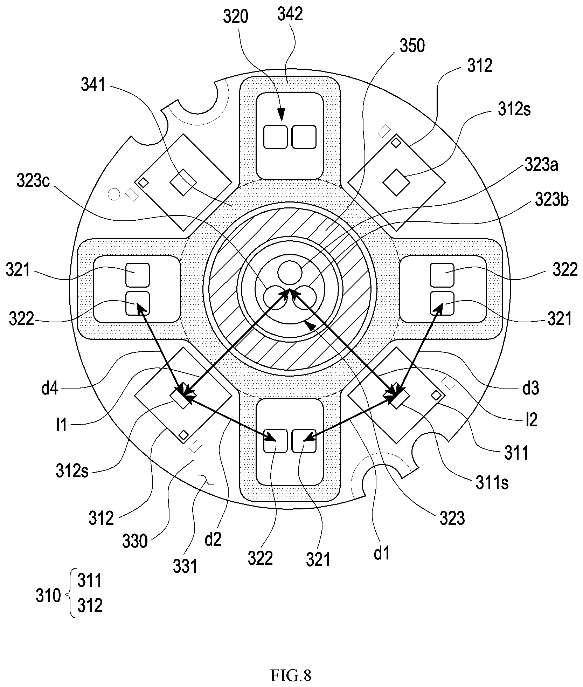

FIG. 8 illustrates distances between light emitters 321 and 322 and photodetectors 311 and 312 in a sensor module 300 according to an embodiment of the disclosure.

Referring to FIG. 8, the second light emission module 323 according to an embodiment of the disclosure may be located on an inner side of the board 330. The first light emitter 321, the second light emitter 322, and/or the photodetectors 311 and 312 may be located on an outer side (e.g., edge) of the board 330. In an example, the second light emission module 323 may be located at a center of the surface 331 of the board 330 and surrounded by a plurality of light emitters 321 and 322 and a plurality of photodetectors 311 and 312.

Accordingly, as the first light emission module 320 configured to emit light (e.g., blue light and UV light) having a relatively short wavelength is disposed closer to the photodetectors 311 and 312 than the second light emission module 323 configured to emit light (e.g., green light, red light, and IR light) having a relatively long wavelength, the photodetectors 311 and 312 may detect the light having the relatively short wavelength more effectively.

According to an embodiment of the disclosure, a distance d1 between a light receiving portion 311s of the first photodetector 311 and a light source of the first light emitter 321 may be smaller than a distance 12 between the light receiving portion 311s of the first photodetector 311 and a light source of the third light emitters 323a, 3223b, and 323c. In an example, the light receiving portion 311s of the first photodetector 311 may be a photosensitive layer of a photodiode.

According to an embodiment of the disclosure, a distance d2 between a light receiving portion 312s of the second photodetector 312 and a light source of the second light emitter 322 may be smaller than a distance 11 between the light receiving portion 312s of the second photodetector 312 and the light source of the third light emitter 323a, 3223b, and 323c. In an example, the light receiving portion 312s of the second photodetector 312 may be a photosensitive layer of a photodiode.

According to an embodiment of the disclosure, when viewed from above the surface 331 of the board 330, the distance d2 or d4 between the light source of the second light emitter 322 and the light receiving portion 312s of the second photodetector 312 may range from 3mm to 4mm. More specifically, it may range from 3mm to 3.5mm.

According to an embodiment of the disclosure, when viewed from above the surface 331 of the board 330, the distance d1 or d3 between the light source of the first light emitter 321 and the light receiving portion 311s of the first photodetector 311 may range from 3mm to 4mm. More specifically, it may range from 3mm to 3.5mm.

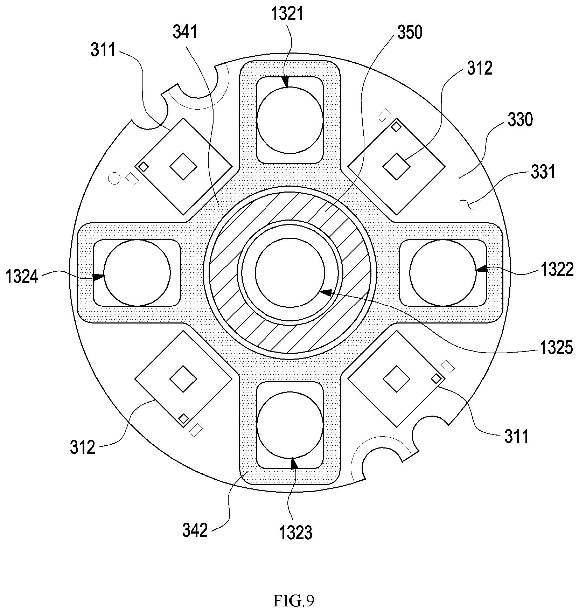

FIG. 9 illustrates a plurality of light emission modules 1321, 1322, 1323, 1324, and 1325 and photodetectors 311 and 312 according to an embodiment of the disclosure.

The description of the components (e.g., the first light emission module 320 and the second light emission module 323) made with reference to FIGS. 6 to 8 is applicable substantially equally to the components (e.g., light emission modules 1321, 1322, 1323, 1324, and 1325) of the same names described with reference to FIG. 9, unless conflicting each other.

Referring to FIG. 9, the wearable device 103 according to an embodiment of the disclosure may include the plurality of light emission modules 1321, 1322, 1323, 1324, and 1325. Each of the light emission modules 1321, 1322, 1323, 1324, and 1325 may correspond to the first light emission module 320 or the second light emission module 323 described with reference to FIG. 6.

According to an embodiment of the disclosure, the wearable device 103 may include a plurality of second light emission modules 323, unlike FIG. 6. In an example, the plurality of second light emission modules 323 may be located at positions corresponding to some 1322, 1324, and 1325 of the light emission modules 1321, 1322, 1323, 1324, and 1325 illustrated in FIG. 9, and a plurality of first light emission modules 320 may be arranged at positions corresponding to the remaining light emission modules 1321 and 1323.

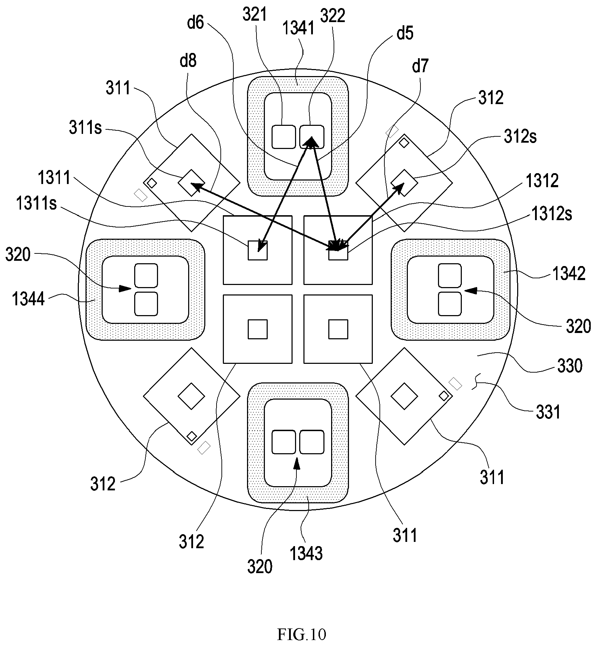

FIG. 10 illustrates first light emission modules 320 and photodetectors 1311 and 1312 according to an embodiment of the disclosure.

Referring to FIG. 10, the wearable device 103 according to an embodiment of the disclosure may include a third photodetector 1311. The description of the first photodetector 311 made with reference to FIG. 6 is applicable substantially equally to the third photodetector 1311, unless conflicting each other. The third photodetector 1311 may be disposed on the inner side of the surface 331 of the board 330. In an example, the third photodetector 1311 may be disposed at the center of the surface 331 of the board 330, surrounded by first photodetectors 311 and second photodetectors 312.

The wearable device 103 according to an embodiment of the disclosure may include a fourth photodetector 1312. The description of the second photodetector 312 given with reference to FIG. 6 is applicable substantially equally to the fourth photodetector 1312, unless conflicting each other. The fourth photodetector 1312 may be disposed on the inner side of the surface 331 of the board 330. In an example, the fourth photodetector 1312 may be disposed at the center of the surface 331 of the board 330, surrounded by the first photodetectors 311 and the second photodetectors 312.

According to an embodiment of the disclosure, the first photodetectors 311, the second photodetectors 312, the first light emitter 321, and the second light emitter 322 may be arranged around the third photodetector 1311 and the fourth photodetector 1312 to at least partially surround the third photodetector 1311 and the fourth photodetector 1312. In an example, the first photodetectors 311, the second photodetectors 312, the first light emitter 321, and the second light emitter 322 may be arranged at radial positions of the third photodetector 1311 and the fourth photodetector 1312.

According to an embodiment of the disclosure, the wearable device 103 may include the third photodetector 1311 configured to detect light having the first wavelength or longer. The wearable device 103 may include the fourth photodetector 1312 configured to detect light having the second wavelength or longer. The third photodetector 1311 and the fourth photodetector 1312 may detect light emitted from the user's skin.

According to an embodiment of the disclosure, when viewed from above the surface 331 of the board 330, the fourth photodetector 1312 may be disposed closer to the second light emitter 322 than to the first light emitter 321. In an example, when viewed from above the surface 331 of the board 330, a distance d5 between the light source of the second light emitter 322 and a light receiving portion 1312s of the fourth photodetector 1312 may be smaller than a distance d6 between the light source of the second light emitter 322 and a light receiving portion 1311s of the third photodetector 1311.

According to an embodiment of the disclosure, when viewed from above the surface 331 of the board 330, the fourth photodetector 1312 may be disposed closer to the second photodetector 312 than to the first photodetector 311. In an example, a distance d7 between the light receiving portion 1312s of the fourth photodetector 1312 and the light receiving portion 312s of the second photodetector 312 may be smaller than a distance d8 between the light receiving portion 1312s of the fourth photodetector 1312 and the light receiving portion 311s of the first photodetector 311.

According to an embodiment of the disclosure, the wearable device 103 may include wall structures 1341, 1342, 1343, and 1344. The wall structures 1341, 1342, 1343, and 1344 may surround a second light emission modules 320. In an example, a plurality of wall structures 1341, 1342, 1343, and 1344 may be provided and surround a plurality of second light emission modules 320, respectively.

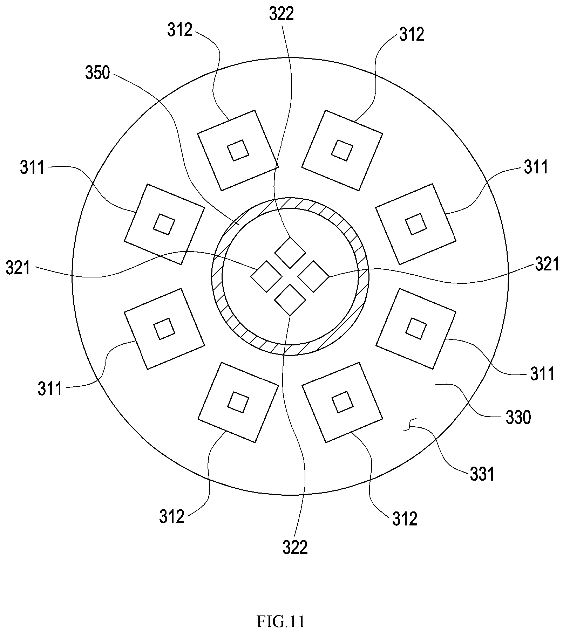

FIG. 11 illustrates light emitters 321 and 322 and photodetectors 311 and 312 according to an embodiment of the disclosure.

Referring to FIG. 11, the first wall 350 may be disposed between the light emitters 321 and 322 and the photodetectors 311 and 312. In an example, the first wall 350 may surround a plurality of light emitters 321 and 322, and a plurality of photodetectors 311 and 312 may surround the first wall 350.

According to an embodiment of the disclosure, an order in which the light emitters 321 and 322 are arranged along a longitudinal direction of the first wall 350 may be identical to an order in which the photodetectors 311 and 312 are arranged along the longitudinal direction of the first wall 350. In an example, the light emitters 321 and 322 may be arranged in the order of the first light emitter 321 and the second light emitter 322 along the longitudinal direction of the first wall 350, and the photodetectors 311 and 312 may be arranged in the order of the first photodetector 311 and the second photodetector 312 along the longitudinal direction of the first wall 350.

According to an embodiment of the disclosure, the first photodetector 311 may be spaced apart from the first light emitter 321 toward the edge of the board 330. In an example, the first photodetector 311 may be spaced apart from the first light emitter 321 in a radial direction of the board 330. The second photodetector 312 may be spaced apart from the second light emitter 322 toward the edge of the board 330. In an example, the second photodetector 312 may be spaced apart from the second light emitter 322 in the radial direction of the board 330.

According to an embodiment of the disclosure, the first photodetectors 311 and the second photodetectors 312 may be arranged around the first light emitter 321 and the second light emitter 322 to at least partially surround the first light emitter 321 and the second light emitter 322. In an example, the first photodetectors 311 and the second photodetectors 312 may be arranged at radial positions of the first light emitter 321 and the second light emitter 322.

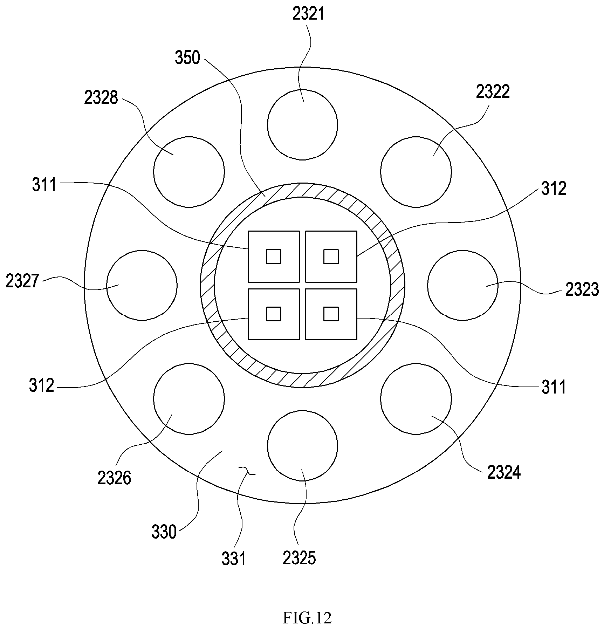

FIG. 12 illustrates light emission modules 2321, 2322, 2323, 2324, 2325, 2326, 2327, and 2328 and photodetectors 311 and 312 according to an embodiment of the disclosure.

The description of the light emission modules 320 and 323 given with reference to FIGS. 6 to 8 is applicable substantially equally to the light emission modules 2321, 2322, 2323, 2324, 2325, 2326, 2327, and 2328 of the same names described with reference to FIG. 12, unless conflicting with each other.

Referring to FIG. 12, the wearable device 103 according to an embodiment of the disclosure may include a plurality of light emission modules 2321, 2322, 2323, 2324, 2325, 2326, 2327, and 2328. Each of the light emission modules 2321, 2322, 2323, 2324, 2325, 2326, 2327, and 2328 may correspond to the first light emission module 320 or the second light emission module 323 described with reference to FIG. 6.

According to an embodiment of the disclosure, the plurality of light emission modules 2321, 2322, 2323, 2324, 2325, 2326, 2327, and 2328 may surround the photodetectors 311 and 312. The photodetectors 311 and 312 may correspond to the first photodetector 311 and the second photodetector 312 described with reference to FIG. 6. Light emitted from the plurality of light emission modules 2321, 2322, 2323, 2324, 2325, 2326, 2327, and 2328 may correspond to the first photodetector 311 and the second photodetector 312.

According to an embodiment of the disclosure, the plurality of light emission modules 2321, 2322, 2323, 2324, 2325, 2326, 2327, and 2328 may be arranged around the photodetectors 311 and 312 to at least partially surround the photodetectors 311 and 312. In an example, the plurality of light emission modules 2321, 2322, 2323, 2324, 2325, 2326, 2327, and 2328 may be arranged on radial positions of the photodetectors 311 and 312.

According to an embodiment of the disclosure, the wearable device 103 may include the first wall 50 disposed between the photodetectors 311 and 312 and the plurality of light emission modules 2321, 2322, 2323, 2324, 2325, 2326, 2327, and 2328. The first wall 350 may surround the photodetectors 311 and 312.

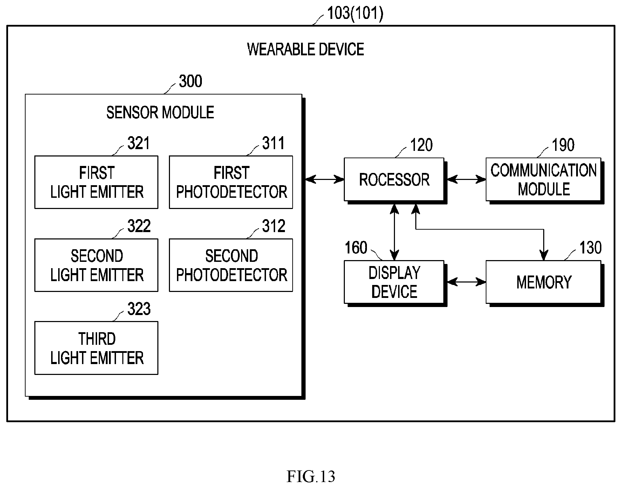

FIG. 13 is a block diagram illustrating a portion of the wearable device 103 according to an embodiment of the disclosure.

Referring to FIG. 13, the wearable device 103 according to an embodiment of the disclosure may include the processor 120, the memory 130, a display device 160 (e.g., the display module 160 in FIG. 1), the sensor module 300 (e.g., the sensor module 176 in FIG. 1 or the sensor module 211 in FIG. 2), and the communication module 190. The processor 120, the memory 130, the display device 160, the sensor module 300, and the communication module 190 may be operatively coupled to an electrical interface such as a communication bus (not shown). The communication module 190 may perform at least one of transmission or reception of a wired or wireless signal including information related to the user's blood sugar level. Each of the light emitters 321, 322, and 323 and the photodetectors 311 and 312 may be controlled by the processor 120.

According to an embodiment of the disclosure, the display device 160 may be controlled by the processor 120 to provide a visual user interface (UI) to the user. For example, the display device 160 may include a display that is at least partially visible to the outside through a housing of the electronic device 101. The display may output information visually to the user using at least one of an organic light emitting diode (OLED), a liquid crystal display (LCD), or a light emitting diode (LED). A UI output through the display may include the amount of AGEs in the body or biometric information (e.g., body age) about the user based the amount of AGEs (see FIG. 4).

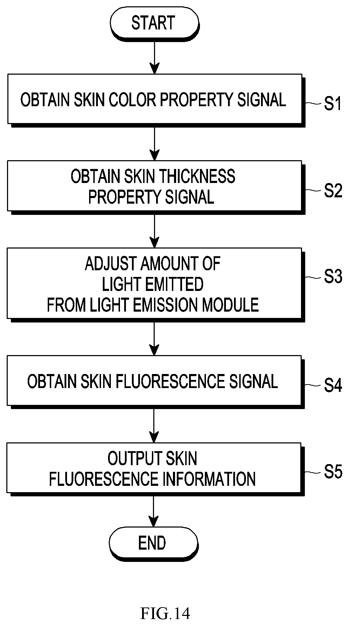

FIG. 14 is a flowchart illustrating a method for measuring skin fluorescence in the wearable device 103according to an embodiment of the disclosure. The method for measuring skin fluorescence illustrated in FIG. 14 may be performed using the wearable device 103described with reference to FIGS. 6 to 12.

Referring to FIGS. 13 and 14, according to an embodiment of the disclosure, the wearable device 103 may include the light emission modules 320 and 323 configured to emit light to the user's skin, and the photodetecting module 310 (see FIG. 6) configured to detect light emitted from the user's skin. The light emission modules 320 and 323 may include the first light emission module 320 and the second light emission module 323 described with reference to FIG. 6. The photodetecting module 310 may include the first photodetector 311 and the second photodetector 312 described with reference to FIG. 6.

The method for measuring skin fluorescence in the wearable device 103 according to an embodiment of the disclosure may include operation S1 for emitting at least one of red light, blue light, or green light to the user's skin through the light emission modules 320 and 323, and obtaining a skin color property signal value through the photodetecting module 310.

According to an embodiment of the disclosure, in operation S1 for obtaining a skin color property signal value, the blue light emitter 321 of the first light emission module 320, and the red light emitter 323b and green light emitter 323c of the second light emission module 323 may operate sequentially in a random order to emit light toward the user's skin. In an example, the blue light emitter 321, the green light emitter 323c, and the red light emitter 323b may sequentially emit light, and light emitted from the user's skin may be detected through the photodetecting module 310 in each operation.

According to an embodiment of the disclosure, the photodetecting module 310 may obtain a signal value corresponding to the detected emitted light. In an example, the first photodetector 311 and/or the second photodetector 312 may obtain a signal value corresponding to the intensity of the detected emitted light, and the processor 120 may determine the skin color of the user's body part (e.g., wrist) by comparing the signal value with a value stored in the memory 130.

According to an embodiment of the disclosure, the method for measuring skin fluorescence in the wearable device 103 may include an operation of emitting UV light to the user's skin by sequentially operating a plurality of UV light emission modules 320, and obtaining a signal value corresponding to each of the light emission modules 320 through the photodetecting module 310, which is performed before operation S1 for obtaining the skin color property signal value. In an example, a signal value corresponding to each of UV light emitters 322 included in a light emission module 320 may be obtained by emitting UV light to the user's skin by sequentially operating the UV light emitters 322 and detecting light emitted from the user's skin through the photodetecting module 310.

According to an embodiment of the disclosure, the processor 120 may determine whether the wearable device 103 is worn based on the signal value corresponding to each of the UV light emitters 322. In an example, when the differences between the signal values corresponding to the respective UV light emitters 322 and a reference value stored in the memory 130 are less than or equal to a reference deviation, the processor 120 may determine that the wearable device 103 is worn properly. Further, when at least one of the differences between the signal values corresponding to the respective UV light emitters 322 and the reference value stored in the memory 130 is greater than the reference deviation, the processor 120 may determine that the wearable device 103 is not worn properly.

According to an embodiment of the disclosure, the method for measuring skin fluorescence in the wearable device 103 may include an operation of notifying whether the device is worn or not. When at least one of the differences between the signal values corresponding to the respective UV light emitters 322 and the reference value stored in the memory 130 is greater than the reference deviation, the wearable device 103 may provide wearable device wearing information to the user. The wearable device wearing information may include information for indicating that the wearable device 103 is not properly worn on the user's body part. In an example, the display device 160 may include the display, and a UI displayed on the display may include the wearable device wearing information. In another example, the wearable device wearing information may be audibly delivered to the user through an audio output module (e.g., the sound output module 155 in FIG. 1) of the wearable device 103. In another example, the wearable device wearing information may be tactilely delivered to the user through a haptic module (e.g., the haptic module 179 in FIG. 1) of the wearable device 103.

The method for measuring skin fluorescence in the wearable device 103 according to an embodiment of the disclosure may include operation S2 for emitting IR light to the user's skin through the light emission modules 320 and 323 and obtaining a skin thickness property signal value through the photodetecting module 310. In operation S2 for obtaining a skin thickness property signal value, IR light may be emitted toward the user's skin through the light emission module 323, and light emitted from the user's skin may be detected by the photodetecting module 310.

According to an embodiment of the disclosure, the photodetecting module 310 may obtain a signal value corresponding to the detected emitted light. In an example, the first photodetector 311 and/or the second photodetector 312 may obtain a signal value corresponding to the intensity of the detected emitted light, and the processor 120 may determine the thickness of the epidermal layer E of the user's body part (e.g., wrist) by comparing the signal value with a value stored in the memory 130.

According to an embodiment of the disclosure, in operation S2 for obtaining a skin thickness property signal value, the illuminance of IR light emitted through the light emission module 323 may correspond to the obtained skin color property signal value. In an example, when the skin color of the user's body part is determined to be a first skin color, IR light with an illuminance corresponding to the first skin color may be emitted in operation S2 for obtaining a skin thickness property signal value. In another example, when the skin color of the user's body part is determined to be a second skin color darker than the first skin color, IR light with an illuminance greater than the illuminance corresponding to the first skin color may be emitted in operation S2 for obtaining a skin thickness property signal value.

The method for measuring skin fluorescence in the wearable device 103 according to an embodiment of the disclosure may include operation S3 for setting an illuminance of UV light emitted through the light emission module 320 based on the obtained skin color property signal value and the obtained skin thickness property signal value.

According to an embodiment of the disclosure, the illuminance of UV light may be set differently depending on the user's skin color in the operation S3 for setting an illuminance of UV light. In an example, when the skin color of the body part is determined to be the first skin color, the illuminance of UV light corresponding to the first skin color may be set in operation S3 for setting an illuminance of UV color. In another example, when the skin color of the user's body part is determined to be the second skin color darker than the first skin color, an illuminance greater than the illuminance corresponding to the first skin color may be set in operation S3 for setting an illuminance of UV color.

According to an embodiment of the disclosure, the illuminance of UV light may be set differently depending on the thickness of the user's epidermis in operation S3 for setting an illuminance of UV color. In an example, when the epidermal thickness of the body part is greater than a predetermined thickness value, the illuminance of UV light may be set to be greater than when the epidermal thickness of the body part is smaller than the predetermined thickness value. In another example, when the epidermal thickness of the body part is smaller than the predetermined thickness value, the illuminance of UV light may be set to be less than when the epidermal thickness of the body part is greater than the predetermined thickness value.

The method for measuring skin fluorescence in the wearable device 103 according to an embodiment of the disclosure may include operation S4 for radiating UV light with the set illuminance to the user's skin through the light emission module 320 and obtaining a skin fluorescence signal value through the photodetecting module 310. In operation S4 for obtaining a skin fluorescence signal value, UV light may be emitted to the user's skin through the light emission module 320, and light emitted from the user's skin may be detected by the photodetecting module 310.

Referring to FIG. 5, according to an embodiment of the disclosure, the UV light UV emitted to the user's skin through the light emission module 320 may reach the dermal layer T of the user's skin and be absorbed by the dermal layer proteins P1, P2, and P3 in which AGEs are combined, and the dermal layer proteins P1, P2, and P3 may emit the light EL in the form of fluorescence. In an example, the wavelength of the UV light UV emitted from the light emission module 320 may be about 365nm, and the wavelength of the emitted light EL from the dermal layer proteins P1, P2, and P3 may be in a band of 500nm.

According to an embodiment of the disclosure, the emitted light EL may be detected through the photodetecting module 310. The photodetecting module 310 may obtain a signal value corresponding to the intensity of the emitted light EL. In an example, the first photodetector 311 and/or the second photodetector 312 may obtain a signal value corresponding to the intensity of the detected emitted light EL, and the processor 120 may determine a skin fluorescence value of the user's body part (e.g., wrist) by comparing the signal value with a skin fluorescence value stored in the memory 130. In an example, the skin fluorescence value may include the amount of the AGEs in the body.

According to an embodiment of the disclosure, the method for measuring skin fluorescence in the wearable device 103 may further include an operation S5 of outputting skin fluorescence information. The skin fluorescence information may include the amount of the AGEs in the body or information (e.g., the user's body age) that may be estimated from the amount of the AGEs.

According to an embodiment of the disclosure, the skin fluorescence information may be visually delivered to the user through the display device 160. In an example, the display device 160 may include the display, and a UI displayed on the display may include the skin fluorescence information. In another example, the skin fluorescence information may be audibly delivered to the user through the audio output module (e.g., the sound output module 155 in FIG. 1) of the wearable device 103. In another example, the skin fluorescence information may be tactilely delivered to the user through the haptic module (e.g., the haptic module 179 in FIG. 1) of the wearable device 103.

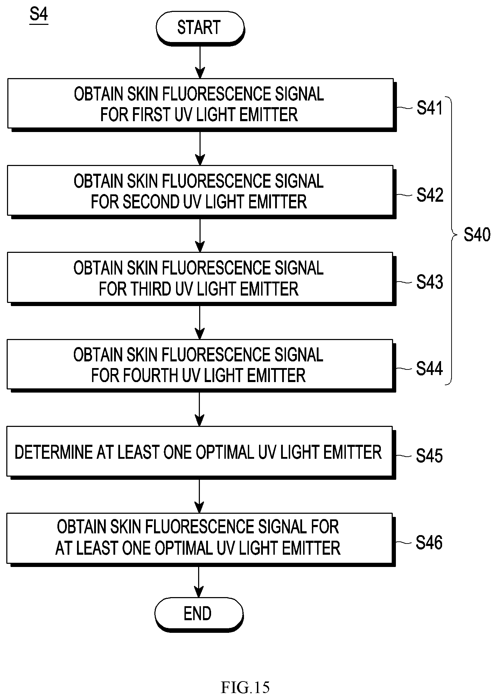

FIG. 15 is a flowchart illustrating operation S4 for obtaining a skin fluorescence signal value according to an embodiment of the disclosure.

Referring to FIG. 15, the light emission modules 320 and 323 according to an embodiment of the disclosure may include a plurality of first light emission modules 320 comprising UV light emitter 322. In an example, the plurality of first light emission modules 320 may be arranged on the surface 331 of the board 330, as illustrated in FIG. 6.

According to an embodiment of the disclosure, operation S4 for obtaining a skin fluorescence signal value may include operation S40 for emitting UV light to the user's skin by sequentially operating the plurality of first light emission modules 320, and obtaining a signal value corresponding to each of the first light emission modules 320 through the photodetectors 311 and 312.

According to an embodiment of the disclosure, operation S40 for obtaining a signal value corresponding to each first light emission module 320 may include operations S41, S42, S43, and S44 for sequentially emitting UV light through a plurality of UV light emitters 322 of each first light emission module 320 and obtaining skin fluorescence signal values through the photodetecting module 310 (see FIG. 6). In an example, the plurality of UV light emitters 322 may correspond to the second light emitters 322 illustrated in FIG. 6, and for convenience of the description made with reference to FIG. 15, the plurality of UV light emitters 322 may be referred to as a first to fourth UV light emitters.

According to an embodiment of the disclosure, operation S40 for obtaining a signal value corresponding to each first light emission module 320 may be performed in the order of operation S41 for obtaining a skin fluorescence signal for the first UV light emitter 322, operation S42 for obtaining a skin fluorescence signal for the second UV light emitter 322, operation S43 for obtaining a skin fluorescence signal for the third UV light emitter 322, and operation S44 for obtaining a skin fluorescence signal for the fourth UV light emitter 322.

According to an embodiment of the disclosure, in operations S41, S42, S43, and S44 for obtaining skin fluorescence signals for the first to fourth UV light emitters 322, the light EL (see FIG. 5) emitted from the user's skin may be detected through the photodetecting module 310. The photodetecting module 310 may obtain a signal value corresponding to the intensity of the emitted light EL. In an example, the first photodetector 311 and/or the second photodetector 312 may obtain a signal value corresponding to the intensity of the detected emitted light EL, and the processor 120 may determine the skin fluorescence value of the user's body part (e.g., wrist) by comparing the signal value with a skin fluorescence value stored in the memory 130. In an example, the skin fluorescence value may include the amount of AGEs in the body.

According to an embodiment of the disclosure, operation S4 for obtaining a skin fluorescence signal value may include operation S45 for determining at least one optimal first light emission module among the plurality of first light emission modules 320 by comparing a signal value corresponding to each of the first light emission modules 320 with a preset value.

According to an embodiment of the disclosure, in operation S45 for determining an optimal first light emission module 320, the processor 120 may determine a UV light emitter 322 having the highest of the skin fluorescence signal values obtained in operations S41, S42, S43, and S44 for obtaining skin fluorescence signal values for the first to fourth UV light emitters 322 as an optimal UV light emitter 322. Additionally, a first light emission module 320 including the optimal UV light emitter 322 may be determined as an optimal first light emission module 320. The UV light emitter 322, which has the highest skin fluorescence signal value, may be understood as a UV light emitter that facilitates measurement of the degree of fluorescence of the user's skin (e.g., the amount of AGEs in the body).

According to an embodiment of the disclosure, operation S4 for obtaining a skin fluorescence signal value may include operation S46 for obtaining a skin fluorescence signal value for the optimal UV light emitter. In operation S46 for obtaining a skin fluorescence signal value for the optimal UV light emitter, UV light directed to the user's skin may be emitted through the optimal UV light emitter, and light EL (see FIG. 5) emitted from the user's skin may be detected through the photodetecting module 310. The first photodetector 311 and/or the second photodetector 312 may obtain a signal value corresponding to the intensity of the detected emitted light EL, and the processor 120 may determine a skin fluorescence value of the user's body part (e.g., wrist) by comparing the signal value with the skin fluorescence value stored in the memory 130.

According to an embodiment of the disclosure, operation S46 of obtaining a skin fluorescence signal value for the optimal UV light emitter may be omitted. The processor 120 may determine the highest of the skin fluorescence signal values obtained in operations S41, S42, S43, and S44 for obtaining skin fluorescence signals for the first to fourth UV light emitters 322 to be the skin fluorescence value of the user's body part (e.g. wrist).

A wearable device is a device worn on a user's body part (e.g., wrist, ankle, or the like), which may detect biometric information about the user through the user's skin or effectively provide information to the user. However, it is difficult to stably fix the wearable device to the user's body part due to the user's physical activity. Accordingly, much research is being conducted to improve biometric information measurement through a wearable device.

An object to be achieved by the disclosure may be to improve the performance of measuring user biometric information (e.g., the amount of AGEs) by a wearable device.

An object to be achieved by the disclosure may be to effectively arrange a plurality of components (e.g., light emitters and photodetectors) for detecting biometric information in a wearable device.

The objects to be addressed by the disclosure are not limited to the above-mentioned objects, and may be determined in various ways without departing from the spirit and scope of the disclosure.

An electronic device according to various embodiments of the disclosure may improve the performance of biometric information measurement of a wearable device by disposing a short-wavelength light emitter closer to a photodetector than a long-wavelength light emitter.

An electronic device according to various embodiments of the disclosure may enable effective arrangement of components in a wearable device by closely arranging a plurality of light emitters that emit light of different wavelengths and a plurality of photodetectors that detect light in different wavelength ranges.

The effects that may be obtained from the disclosure are not limited to the effects mentioned above, and other effects not mentioned may be clearly understood by those skilled in the art from the aforementioned description.

The wearable device 103 according to an embodiment of the disclosure may include the housing 210 including the light transmission area 207t.

The wearable device 103 according to an embodiment of the disclosure may include the board 330 disposed inside the housing 210 and including the surface 331 directed toward the light transmission area 207t.

The wearable device 103 according to an embodiment of the disclosure may include the first photodetector 311 disposed on the surface 331 of the board 330 and configured to detect light having a wavelength equal to or longer than a first wavelength.

The wearable device 103 according to an embodiment of the disclosure may include the second photodetector 312 disposed on the surface 331 of the board 330 and spaced apart from the first photodetector 311.

The second photodetector 312 according to an embodiment of the disclosure may be configured to detect light having a wavelength equal to or longer than a second wavelength longer than the first wavelength.

The wearable device 103 according to an embodiment of the disclosure may include the first light emitter 321 disposed between the first photodetector 311 and the second photodetector 312 and configured to emit light having a third wavelength toward the light transmission area 207t.

The wearable device 103 according to an embodiment of the disclosure may include the second light emitter 322 disposed between the first light emitter 321 and the second photodetector 312 and configured to emit light having a fourth wavelength shorter than the third wavelength toward the light transmission area 207t.

The wearable device 103 according to an embodiment of the disclosure may include the light emission module 323 configured to emit light having a fifth wavelength longer than the third wavelength toward the light transmission area 207t.

According to an embodiment of the disclosure, the first photodetector 311, the second photodetector 312, the first light emitter 321, and the second light emitter 322 may be arranged around the light emission module 323 to at least partially surround the light emission module 323.

According to an embodiment of the disclosure, the light emission module 323 may include at least one of the IR light emitter 323a configured to emit IR light, the red light emitter 323b configured to emit red light, or the green light emitter 323c configured to emit green light.

The wearable device 103 according to an embodiment of the disclosure may further include the walls 341 and 350 surrounding the light emission module 323 and protruding from the surface 331 of the board 330 toward the light transmission area 207t.

According to an embodiment of the disclosure, the first photodetector 311, the first light emitter 321, the second light emitter 322, and the second photodetector 312 may be sequentially arranged along an edge of the light transmission area 207t.

The wearable device 103 according to an embodiment of the disclosure may include the wall 342 disposed between the second photodetector 312 and the second light emitter 322 and protruding from the surface 331 of the board 330 toward the light transmission area 207t.

According to an embodiment of the disclosure, the first light emitter 321 may be configured to emit blue light, and the second light emitter 322 may be configured to emit UV light.

According to an embodiment of the disclosure, the wearable device 103 may include the third light emitters 323a, 323b, and 323c configured to emit light having a wavelength equal to or longer than the third wavelength toward the light transmission area 207t.

According to an embodiment of the disclosure, the wearable device 103 may include the wall 341 disposed between the third light emitters 323a, 323b, and 323c and the first photodetector 311 and protruding from the surface 331 of the board 330 toward the light transmission area 207t.

According to an embodiment of the disclosure, the distance between the light receiving portion 311s of the first photodetector 311 and the light source of the third light emitters 323a, 323b, and 323c may be greater than the distance between the light receiving portion 312s of the second photodetector 312 and the light source of the second light emitter 322.

According to an embodiment of the disclosure, when viewed from above the surface 331 of the board 330, the distance between the light source of the second light emitter 322 and the light receiving portion 312s of the second photodetector 312 may range from 3mm to 4mm.

According to an embodiment of the disclosure, when viewed from above the surface 331 of the board 330, the distance d1 between the light source of the first light emitter 321 and the light receiving portion 311s of the first photodetector 311 may range from 3mm to 4mm.

According to an embodiment of the disclosure, the second light emitter 322 may include a plurality of second light emitters 322 arranged along an edge of the light transmission area 207t.

According to an embodiment of the disclosure, the wearable device 103 may further include the photodetecting module 1311 and 1312 disposed on the surface 331 of the board 330 and configured to detect light having a specific wavelength.

According to an embodiment of the disclosure, the first photodetector 311, the second photodetector 312, the first light emitter 321, and the second light emitter 322 may be arranged around the photodetecting module 1311 and 1312 to at least partially surround the photodetecting module 1311 and 1312.

According to an embodiment of the disclosure, the photodetecting module 1311 and 1312 may include the third photodetector 1311 configured to detect light having a wavelength equal to or longer than the first wavelength.

According to an embodiment of the disclosure, the photodetecting module 1311 and 1312 may include the fourth photodetector 1312 configured to detect light having a wavelength equal to or longer than the second wavelength.

According to an embodiment of the disclosure, when viewed from above the surface 331 of the board 330, the fourth photodetector 1312 may be disposed to be closer to the second light emitter 322 than to the first light emitter 321.

According to an embodiment of the disclosure, a method for measuring skin fluorescence in the wearable device 103 may include operation S1 for emitting at least one of red light, blue light, or green light toward the user's skin through the light emission modules 320 and 323 and obtaining a skin color property signal value through the photodetecting module 310.

According to an embodiment of the disclosure, the method for measuring skin fluorescence in the wearable device 103 may include operation S2 for emitting IR light toward the user's skin through the light emission modules 320 and 323 and obtaining a skin thickness property signal value through the photodetecting module 310.

According to an embodiment of the disclosure, the method for measuring skin fluorescence in the wearable device 103 may include setting an illuminance of UV light emitted through the light emission modules 320 and 323 based on the obtained skin color property signal value and the obtained skin thickness property signal value.

According to an embodiment of the disclosure, the method for measuring skin fluorescence in the wearable device 103 may include emitting UV light with the set illuminance toward the user's skin through the light emission module 320 and obtaining a skin fluorescence signal value through the photodetecting module 310.

According to an embodiment of the disclosure, the light emission modules 320 and 323 may include a plurality of first light emission modules 320 including UV light emitters 322.

According to an embodiment of the disclosure, operation S4 for obtaining the skin fluorescence signal value may include emitting UV light through an optimal first light emission module 320 among the plurality of first light emission modules 320.

According to an embodiment of the disclosure, operation S4 for obtaining the skin fluorescence signal value may include operations S41, S42, S43, and S44 for emitting UV light toward the user's skin by sequentially operating the plurality of first light emission modules 320 and obtaining a signal value corresponding to each of the first light emission modules 320 through the photodetecting module 310.

According to an embodiment of the disclosure, operation S4 for obtaining the skin fluorescence signal value may include operation S45 for determining the optimal first light emission module 320 among the plurality of first light emission modules 320 by comparing the signal value corresponding to each of the first light emission modules 320 with a preset value.

According to an embodiment of the disclosure, operation S2 for obtaining the skin thickness property signal value may include setting an illuminance of the IR light emitted through the light emission module 323 based on the obtained skin color property signal value.

According to an embodiment of the disclosure, the method for measuring skin fluorescence in the wearable device 103 may further include, before operation S1 for obtaining the skin color property signal value, emitting UV light toward the user's skin by sequentially operating the plurality of first light emission modules 320 and obtaining a signal value corresponding to each of the first light emission modules 320 through the photodetecting module 310.

According to an embodiment of the disclosure, the method for measuring skin fluorescence in the wearable device 103 may further include, before operation S1 for obtaining the skin color property signal value, when a difference between the signal value corresponding to each of the first light emission modules 320 and a predetermined reference value is greater than a reference deviation, providing wearable device wearing information visually or audibly through the wearable device 103.

While the disclosure has been shown and described with reference to various embodiments thereof, it will be understood by those skilled in the art that various changes in form and details may be made therein without departing from the spirit and scope of the disclosure as defined by the appended claims and their equivalents.

## Claims

1. A wearable device(103) comprising:
a housing(210) including a light transmission area(207t);
a board(330) disposed inside the housing(210) and including a surface(331) directed toward the light transmission area(207t);
a first photodetector(311) disposed on the surface(331) of the board(330) and configured to detect light having a wavelength equal to or longer than a first wavelength;
a second photodetector(312) disposed on the surface(331) of the board(330) and configured to detect light having a wavelength equal to or longer than a second wavelength longer than the first wavelength;
a first light emitter(321) disposed between the first photodetector(311) and the second photodetector(312) and configured to irradiate light having a third wavelength toward the light transmission area(207t); and
a second light emitter(322) disposed between the first light emitter(321) and the second photodetector(312) and configured to emit light having a fourth wavelength shorter than the third wavelength toward the light transmission area(207t).

2. The wearable device of claim 1, further comprising:
a light emission module(323) configured to emit light having a fifth wavelength longer than the third wavelength toward the light transmission area(207t),
wherein the first photodetector(311), the second photodetector(312), the first light emitter(321), and the second light emitter(322) are arranged around the light emission module(323) to at least partially surround the light emission module (323).

3. The wearable device of claim 1 or 2, wherein the light emission module(323) includes at least one of an infrared (IR) light emitter(323a) configured to emit IR light, a red light emitter(323b) configured to emit red light, or a green light emitter(323c) configured to emit green light.

4. The wearable device of any one of claims 1 to 3, further comprising:
a wall(341, 350) surrounding the light emission module(323) and protruding from the surface(331) of the board(330) toward the light transmission area(207t).

5. The wearable device of any one of claims 1 to 4, wherein the first photodetector, the first light emitter, the second light emitter, and the second photodetector are sequentially arranged along an edge of the light transmission area.

6. The wearable device of any one of claims 1 to 5, further comprising:
a wall disposed between the second photodetector and the second light emitter and protruding from the surface of the board toward the light transmission area.

7. The wearable device of any one of claims 1 to 6,
wherein the first light emitter is configured to emit blue light, and
wherein the second light emitter is configured to emit ultraviolet (UV) light.

8. The wearable device of any one of claims 1 to 7, further comprising:
a third light emitter configured to emit light having a wavelength equal to or longer than the third wavelength toward the light transmission area.

9. The wearable device of any one of claims 1 to 8, further comprising:
a wall disposed between the third light emitter and the first photodetector and protruding from the surface of the board toward the light transmission area.

10. The wearable device of any one of claims 1 to 9, wherein a distance between a light receiving portion of the first photodetector and a light source of the third light emitter is greater than a distance between a light receiving portion of the second photodetector and a light source of the second light emitter.

11. The wearable device of any one of claims 1 to 10, wherein when viewed from above the surface of the board, a distance between a light source of the second light emitter and a light receiving portion of the second photodetector ranges from 3mm to 4mm.

12. The wearable device of any one of claims 1 to 11, wherein when viewed from above the surface of the board, a distance between a light source of the first light emitter and a light receiving portion of the first photodetector ranges from 3mm to 4mm.

13. The wearable device of any one of claims 1 to 12, wherein the second light emitter includes a plurality of second light emitters arranged along an edge of the light transmission area.

14. The wearable device of any one of claims 1 to 13, further comprising:
a third photodetector disposed on the surface of the board and configured to detect light having a wavelength equal to or longer than the first wavelength; and
a fourth photodetector disposed on the surface of the board and configured to detect light having a wavelength equal to or longer than the second wavelength,
wherein the first photodetector, the second photodetector, the first light emitter, and the second light emitter are arranged around the third photodetector and the fourth photodetector to at least partially surround the third photodetector and the fourth photodetector.

15. The wearable device of any one of claims 1 to 14, wherein when viewed from above the surface of the board, the fourth photodetector is disposed to be closer to the second light emitter than to the first photodetector.
